# EUROPEAN PATENT APPLICATION

(11) **EP 4 385 534 A1**
(43) Date of publication of application: **19.06.2024**
(21) Application number: 22213277.1
(22) Date of filing: 13.12.2022
(51) Int. Cl.: A61M 1/00

(54) **APPARATUSES AND METHODS FOR NEGATIVE PRESSURE WOUND THERAPY**

(71) Applicant: Mölnlycke Health Care AB, 402 52 Göteborg (SE)
(72) Inventor: RODZEWICZ, Patrick, 412 52 Göteborg (SE)
(74) Representative: Zacco Sweden AB

(57) **Abstract**

An apparatus and related aspects for providing reduced pressure to a tissue site are disclosed. The apparatus comprises a source of negative pressure configured to provide negative pressure via a fluid flow path from an inlet of the source of negative pressure to a wound dressing. The apparatus further comprises control circuitry configured to control operation of the source of negative pressure. The control circuitry is further configured to generate a signal so to cause the apparatus or an external handheld device to emit a signal comprising an audible sound and/or a tactile signal to a user of the apparatus during a time period prior to activation of the source of negative pressure.

## Description

### TECHNICAL FIELD

The herein disclosed embodiments generally relate to negative pressure wound therapy (NPWT). In particular, the herein disclosed embodiments relate to apparatuses for providing reduced pressure to a tissue site, methods for controlling an apparatus for providing reduced pressure to a tissue site, and thereto related computer program products and computer-readable storage media.

### BACKGROUND

Negative pressure wound therapy (NPWT) is a technique that promotes healing of e.g. surgical, acute and chronic wounds by the application of a sub-atmospheric pressure ("negative pressure") to the wound, using a negative pressure pump. Wound healing is achieved by applying a negative pressure, such as "vacuum" through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

Generally, the NPWT technique has until now mainly been applied to a patient while in a hospital environment. However, recent product development now allows the technique to be used by a patient in a home environment.

Introducing the NPWT device in the home environment may in some situations have further implications on the patient and/or e.g. a partner of the patient. Specifically, when the negative pressure pump is operating, the pump may generate noise that may be perceived as disturbing for the patient and/or the partner of the patient.

### SUMMARY

The herein disclosed technology seeks to mitigate, alleviate or eliminate one or more of the above-identified deficiencies and disadvantages in the prior art to address various problems relating to discomfort or disturbances caused by the noise generated by the NPWT apparatus during normal operation.

Various aspects and embodiments of the disclosed technology are defined below and in the accompanying independent and dependent claims.

An aspect of the disclosed technology comprises an apparatus for providing reduced pressure to a tissue site. The apparatus comprises a source of negative pressure configured to provide negative pressure via a fluid flow path from an inlet of the source of negative pressure to a wound dressing. The apparatus further comprises control circuitry configured to control operation of the source of negative pressure. The control circuitry is further configured to generate a signal so to cause the apparatus or an external handheld device to emit a signal comprising an audible sound and/or a tactile signal to a user of the apparatus during a time period prior to activation of the source of negative pressure.

Another aspect of the disclosed technology comprises a method for controlling an apparatus comprising a source of negative pressure configured to provide negative pressure via a fluid flow path from an inlet of the source of negative pressure to a wound dressing. The method comprises generating a signal so to cause the apparatus or an external handheld device to emit a signal comprising an audible sound and/or a tactile signal to a user of the apparatus during a time period prior to activation of the source of negative pressure. With this aspect of the disclosed technology, similar advantages and preferred features are present as in the other aspects.

Another aspect of the disclosed technology comprises a computer program product comprising instructions which, when the program is executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

Another aspect of the disclosed technology comprises a (non-transitory) computer-readable storage medium comprising instructions which, when executed by a computing device of an apparatus for providing reduced pressure to a wound dressing, causes the apparatus to carry out the method according to any one of the embodiments disclosed herein. With this aspect of the disclosed technology, similar advantages and preferred features are present as in the other aspects.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

The disclosed aspects and preferred embodiments may be suitably combined with each other in any manner apparent to anyone of ordinary skill in the art, such that one or more features or embodiments disclosed in relation to one aspect may also be considered to be disclosed in relation to another aspect or embodiment of another aspect.

An advantage of some embodiments is that the reactions to the sounds made by the pump of an NPWT apparatus may be reduced.

An advantage of some embodiments is that patient discomfort due to unpleasant noise caused by the pump motor of an NPWT apparatus may be reduced.

An advantage of some embodiments is that the sleep quality for patients wearing an NPWT system while sleeping may be improved.

Further embodiments are defined in the dependent claims. It should be emphasized that the term "comprises/comprising" when used in this specification is taken to specify the presence of stated features, integers, steps, or components. It does not preclude the presence or addition of one or more other features, integers, steps, components, or groups thereof.

These and other features and advantages of the disclosed technology will in the following be further clarified with reference to the embodiments described hereinafter.

### BRIEF DESCRIPTION OF THE DRAWINGS

The above aspects, features and advantages of the disclosed technology, will be more fully appreciated by reference to the following illustrative and non-limiting detailed description of example embodiments of the present disclosure, when taken in conjunction with the accompanying drawings, in which:
Fig. 1 is a schematic illustration of a wound treatment system in accordance with some embodiments.
Fig. 2 is a schematic illustration of a wound treatment system in accordance with some embodiments.
Fig. 3a is a schematic illustration of a subject's startle response in reference to a startle stimulus.
Fig. 3b is a schematic illustration of a subject's startle response in reference to a startle stimulus that is preceded by a prepulse.
Fig. 4 is a schematic flowchart representation of a method for controlling an apparatus of a wound treatment system in accordance with some embodiments.

### DETAILED DESCRIPTION

The present disclosure will now be described in detail with reference to the accompanying drawings, in which some example embodiments of the disclosed technology are shown. The disclosed technology may, however, be embodied in other forms and should not be construed as limited to the disclosed example embodiments. The disclosed example embodiments are provided to fully convey the scope of the disclosed technology to the skilled person. Like reference characters refer to like elements throughout. Those skilled in the art will appreciate that the steps, services and functions explained herein may be implemented using individual hardware circuitry, using software functioning in conjunction with hardware circuitry such as a programmed microprocessor or general purpose computer, using one or more Application Specific Integrated Circuits (ASICs), using one or more Field Programmable Gate Arrays (FPGA) and/or using one or more Digital Signal Processors (DSPs).

It will also be appreciated that when the present disclosure is described in terms of a method, it may also be embodied in an apparatus comprising one or more processors, one or more memories coupled to the one or more processors, where computer code is loaded to implement the method. For example, the one or more memories may store one or more computer programs that cause the apparatus to perform the steps, services and functions disclosed herein when executed by the one or more processors in some embodiments.

It is also to be understood that the terminology used herein is for purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. Similarly, "at least one of A and B" is to be interpreted as only A, only B, or both A and B.

It will also be understood that, although the term first, second, etc. may be used herein to describe various elements or features, these elements should not be limited by these terms. These terms are only used to distinguish one element from another. For example, a first signal could be termed a second signal, and, similarly, a second signal could be termed a first signal, without departing from the scope of the embodiments. The first signal and the second signal are both signals, but they are not the same signal.

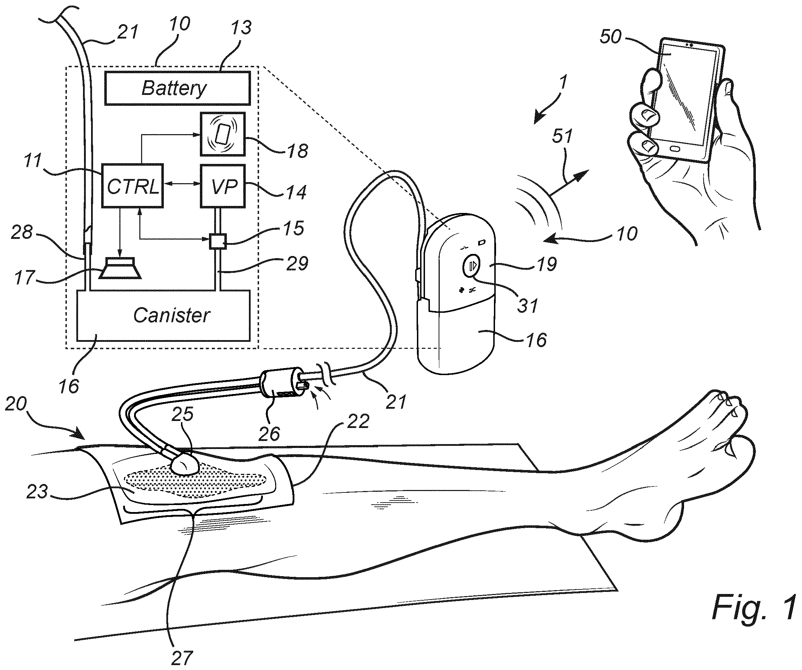

Turning now to the drawings, and to Fig. 1 in particular, there is schematically illustrated a wound treatment system 1 in accordance with some embodiments. The wound treatment system 1 comprises an apparatus 10 for providing reduced pressure to tissue site (or otherwise referred to as a wound site 27). The wound treatment system 1 further comprises a wound cover 22 that is adapted to create a sealed space 23 defined in part by a wound surface, such as at the skin of a user/person, at or around a wound of the user/person. Further, the apparatus 10 (may also be referred to as an NPWT device 10) is fluidly connected to the wound cover 22 using e.g. a tubing 21. The tubing 21 may be of any suitable flexible tubing fabricated from elastomeric or polymeric materials. The tubing 21 may connect to the wound dressing 20, or more specifically to the wound cover 22 of the wound dressing 20, via a suitable connector 25. The connector 25 may be adhered or otherwise attached to the wound cover 22. In particular, the connector 25 may be attached around an opening (not shown) formed in the wound cover 22.

The expression "wound cover" as used herein should be interpreted broadly as any wound site member, e.g. it can be a film sealed around periphery of a wound site 27, where a wound filler may be used to fill the wound volume prior to the application of such wound cover, and/or wound cover may refer to a backing layer of a wound dressing 20 comprising an additional layer(s) such as for example an absorbent layer.

The apparatus 10 comprises a source of negative pressure 14 configured to provide negative pressure (i.e. sub-atmospheric pressure) via a fluid flow path from an inlet of the source of negative pressure 14 to the wound dressing 20. The source of negative pressure 14 is indicated as "VP" ("Vacuum pump") in the figures. In some embodiments, the source of negative pressure 14 comprises a negative pressure pump that together with a motor is adapted for establishing a negative pressure when the source of negative pressure 14 is operating, i.e. when it is in an active state or simply "active". The source of negative pressure may comprise any type of pump and motor that are biocompatible and maintain or draw adequate and therapeutic vacuum levels. Preferably, the negative pressure level to be achieved is in a range between about -20 mmHg and about -300 mmHg. In some embodiments, a negative pressure range between about -80 mmHg and about -140 mmHg is used. In some embodiments, the negative pressure pump is a diaphragm pump, a peristaltic pump, or the like, in which a motor causes the moving parts to draw fluid from the wound site 27.

In the context of the present disclosure, it should be understood that the expressions "negative pressure", "sub- atmospheric pressure", "reduced pressure", or even "vacuum", as used interchangeably herein, generally refer to a pressure less than a local ambient pressure, such as the ambient pressure in a local environment external to a sealed therapeutic environment provided by a wound cover or dressing 22. In many cases, the local ambient pressure may also be the atmospheric pressure at which a patient is located. Unless otherwise indicated, values of pressure stated herein are gauge pressures. Similarly, references to increases in negative pressure typically refer to a decrease in absolute pressure, while decreases in negative pressure typically refer to an increase in absolute pressure.

Further, in some embodiments the apparatus 10 comprises a canister 16 fluidly connected to the negative pressure source 14. The canister 16 may be formed from e.g. moulded plastic or the like, and possibly be a detachable component of the apparatus 10. Moreover, the canister 16 may further be at least partly transparent/translucent to permit viewing into the interior of the canister 16 to assist the user or a caregiver in determining the remaining capacity of the canister 16. As used herein, the term "fluidly connected" should be interpreted broadly and may comprise e.g. any form of tubing, conduits, or channels providing a fluid connection/communication between the canister 16 and the negative pressure source 14 and the dressing 20.

In some embodiments, the canister 16 comprises an inlet port 28 for allowing connection to the tubing 21. The inlet port 28 may also be formed elsewhere at the apparatus 10, however still fluidly connected to the canister 16. The connection between the inlet port 28 and the tubing 21 is a sealed connection, thus ensuring that no leakage is formed at the inlet port 28 during normal operation of the apparatus 10. The tubing 21 is preferably detachably connected to the inlet port 28 through conventional means including a friction fit, bayonet coupling, snap fit, barbed connector, or the like. The inlet port 28 may be moulded/formed from the same material and/or at the same time as forming the canister 16. A similar sealed connection (e.g. using a flange insulation/"O-ring") is formed between the canister 16 (at the outlet port 29) and the source of negative pressure 14.

In some embodiments, the canister 16 is detachably connected to a housing 19 comprising the negative pressure source 14, whereby e.g. a full canister may be removed and replaced with an empty (new) canister. In such embodiments it may be desirable to provide e.g. the canister 16 and the housing 19 with some form of engagement means for securing the canister 16 to the housing such that the canister 16 is not unintentionally removed from the housing 19. The engagement means may in one embodiment comprise a pair of flexible protrusions extending from the canister 16 and adapted to engage with e.g. corresponding locking grooves provided at the housing 19.

However, in some embodiments, the wound treatment system 1 is a canister-less wound treatment system (not shown), where the wound exudate is collected in an absorbent layer, or the like, of the wound dressing 20. In such embodiments, the fluid flow path from the wound dressing 20 to the negative pressure source 14 is provided with one or more suitable filters (not shown) that are adapted to allow gas flow from the wound site 27 to the source of negative pressure 14 and block the flow of liquids from the wound site 27 to the source of negative pressure 14, as readily understood by the person skilled in the art.

In some embodiments, the apparatus 10 comprises a power source, such as e.g. a battery 13 for powering the apparatus 10. The battery 13 may preferably be of the rechargeable type but may alternatively be arranged to be disposable and thus to be changed once discharged. A specifically adapted battery pack may be used in relation to some embodiments.

The apparatus 10 further comprises control circuitry 11 (may also be referred to as "a control unit", "a controller", or the like) electrically connected to the battery 13 and the source of negative pressure 14. The control circuitry 11 is configured to control an operation of the source of negative pressure 14. The control circuitry 11 may comprise a microprocessor, microcontroller, programmable digital signal processor or another programmable device. The control circuitry 11 may also, or instead, include an application specific integrated circuit (ASIC), a programmable gate array or programmable array logic, a programmable logic device, or a digital signal processor. Where the control circuitry 11 includes a programmable device such as the microprocessor, microcontroller or programmable digital signal processor mentioned above, the control circuitry 11 may further include computer executable code that controls operation of the programmable device. Thus, in some embodiments, the control circuitry 11 comprises one or more processors and a memory, where the memory contains instructions executable by the processor whereby the apparatus 10 is operative to execute any one of the method steps or functions disclosed herein.

Furthermore, in some embodiments, the apparatus 10 comprises one or more pressure sensors 15 arranged in fluid connection with the inlet of the source of negative pressure 14, the canister 16, and/or the sealed space 23. In the depicted embodiment of Fig. 1, the apparatus 10 comprises a pressure sensor 15 arranged in a fluid flow path between the canister 16 and the source of negative pressure 14. However, as readily understood by a person skilled in the art, the pressure sensor 15 may be located at any other suitable location to sense or detect a pressure within the fluid flow path between the source of negative pressure 14 and the wound site. For example, the pressure sensor 15 may be arranged within the canister or under the wound cover 22.

During use of the apparatus 10, the wound cover 22 is arranged at a wound site 27 of the user/patient, forming the sealed space 23. The tubing 21 is provided to fluidly connect the wound cover 22 to the inlet port 28 of the apparatus 10. The apparatus 10 is then activated, e.g. by a user or a caregiver pressing a start/pause button 31. The source of negative pressure 14 is thereby activated. When activated, the source of negative pressure 14 will start to evacuate air through the canister 16, the inlet port 28, the tubing 21 and the sealed space 23 formed by the wound cover 22. Accordingly, a negative pressure will be created within the sealed space 23. In case a liquid has been formed at the wound site 27, this liquid from the wound site 27 may at least partly be "drawn" from the wound site, through the tubing 21, the inlet port 28 and into the canister 16. The amount of liquid (possibly defined as exudate) that is drawn from the wound and collected in the canister 16 will depend on the type of wound that is being treated as well as the type of wound dressing 20 used. For example, in case an absorbent dressing is used, the liquid may be absorbed and collected both in the canister 16 and the wound dressing 20, whereas if a dressing with no absorption capacity or little absorption capacity is used, most or all of the liquid from the wound site 27 may be collected in the canister 16. A suitable filter member (not shown) is generally arranged between at the outlet port 29 of the canister 16 to ensure that no liquid is allowed to pass to the source of negative pressure 14 from the canister 16.

In some embodiments, the wound treatment system 1 further comprise an air inlet port 26 or a controlled leakage port 26 that provides a small and controlled inflow of air (as indicated by the arrows) under the wound cover 22 . The air inlet port 26 may for example be provided via a secondary lumen extending from the connector 25 as schematically illustrated. However, in some embodiments the air inlet port 26 may additionally or alternatively be provided at the connector 25 and/or at any suitable location of the tubing 21.

Accordingly, the wound treatment system 1 may in some embodiments be arranged to ensure that a small leakage is present, ensuring that a flow from the sealed space 23 may be achieved without any general blocking e.g. within the tubing 21. Additionally, during use of the wound treatment system 1 some leakage may be expected in relation to the wound cover 22. In some embodiments, the air inlet port 26 includes means for supplying/providing air to the wound site 27 at a predetermined supply rate, the rate being from 2 to 7 ml/min, preferably from 3 to 5 ml/min at a predetermined level of negative pressure. The predetermined level of negative pressure may be from -80 to -180 mmHg, preferably from -100 to -150 mmHg, more preferably from -110 to -140 mmHg. The means for providing air to the wound site 27 (i.e. under the wound cover 22) at a predetermined supply rate may for example be in the form of an air filter (not shown).

In some embodiments, the air filter comprises a hydrophobic and porous material, where the size of the pores is within the range of from 2 to 20 µm, preferably in the range of from 5 to 12 µm. The pore size of the filter is measured in a non-compressed state. In some embodiments, the air filter comprises polyethylene or sintered polyethylene.

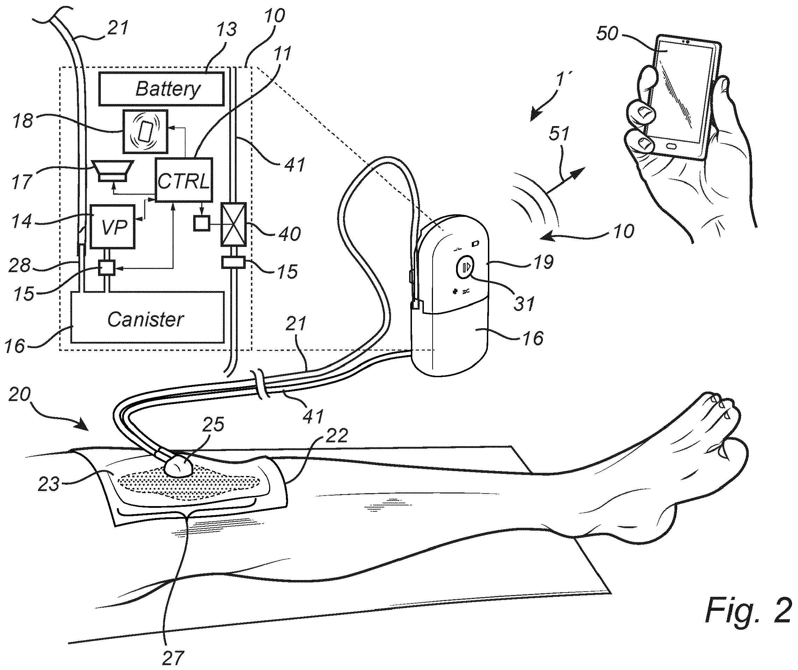

Turning to Fig. 2, a wound treatment system 1' in accordance with some embodiments is schematically illustrated. Many functions, features and components of the wound treatment system 1' depicted in Fig. 2 are the same as or analogous to the wound treatment system 1 depicted in Fig. 1 and will for the sake of brevity and conciseness not be repeated. Instead, the focus will be on the differentiating functions, features, or components between the two wound treatment systems. Thus, as the person skilled in the art readily understands, unless specifically indicated, the foregoing discussion related to the components of the wound treatment system 1 with reference to Fig. 1 is applicable for the corresponding components depicted in Fig. 2 as indicated by the reference numerals.

In general, the wound treatment system 1 depicted in Fig. 1 differs from the wound treatment system 1' depicted in Fig. 2 in that the former is often referred to as a "single-lumen" NPWT system 1 while the latter is often referred to as a "dual-lumen" NPWT system 1'. Accordingly, while the wound treatment system 1 depicted in Fig. 1 has a "controlled inflow of air" (also referred to as a controlled leakage), the wound treatment system 1' depicted in Fig. 2 explicitly controls or regulates the air supply to the wound site 27 by means of the apparatus 10.

Therefore, in contrast to the wound treatment system 1 depicted in Fig. 1, the wound treatment system 1' of Fig. 2 comprises a second fluid flow path that fluidly connects the sealed space 23 created by the wound cover 22 to the ambient atmosphere or a "fluid reservoir" (not shown) via an electronically controllable valve 40. The second fluid flow path (may also be referred to as "air lumen") is defined by a second tubing 41 that may be of any suitable flexible tubing fabricated from elastomeric or polymeric materials. Here, the fluid flow path from the inlet of the source of negative pressure 14 to the wound dressing 20 may be construed as a "first" fluid flow path.

Here, the control circuitry 11 is electronically connected to the electronically controllable valve 40, and the control circuitry 11 is further configured to control an operation of the electronically controllable vale 40 so to release negative pressure from the wound site via the second fluid flow path (or "air lumen") 41. In other words, the control circuitry 11 is configured to open the electronically steerable valve 40 so to introduce fluid (e.g. air) to the wound site 27 when there is a sub-atmospheric pressure under the wound cover 22. This operation may also be referred to as "flushing". Since the wound treatment system 1' depicted in Fig. 2 does not have any "controlled leakage flow", there is a risk that the apparatus 10 would not be able to draw any, or at least a sub-optimal amount of "exudate" from the wound site 27 to the canister 16 once negative pressure has been established under the wound cover 22 due to lack of airflow through the system 1'. Therefore, in order to be able to draw wound exudate from the wound site, a fluid, such as e.g. air from the ambient atmosphere, is controllably introduced at defined time intervals or in response to pressure measurements by opening the electronically steerable valve 40. Once enough fluid has been introduced (e.g. in response to a negative pressure within the first and/or second fluid flow path reaching a lower threshold), the control circuitry 11 is configured to close the electronically steerable valve 40 and to activate the source of negative pressure 14.

The ability to controllably "flush" the system 1' by injecting air via an electronically steerable valve 40 may provide the advantage of longer pressure regulation cycles, which reduces the on-time for the source of negative pressure 11 and therefore reduces the energy consumption of the apparatus 11.

Moving on, regardless of the wound treatment system 1, 1' being single lumen (Fig. 1) or dual lumen (Fig. 2), when the source of negative pressure 14 is activated, either manually for starting treatment (i.e. first depressurization cycle) or automatically during pressure regulation, it produces some relatively uncomfortable noise for the user/patient. In particular when the source of negative pressure 14 has a negative pressure pump and motor that emit a high pitch sound when active. Typically, negative pressure pumps and motors used for NPWT have a high incline and high pitch "sound profile", which "startles" the user/patient and/or any person in close proximity to the apparatus 10 when the source of negative pressure 14 is activated to provide negative pressure. In particular during pressure regulation, where the apparatus 10 automatically controls the on and off periods of the source of negative pressure 14 so to maintain a defined reduced pressure level at the wound site 23, the user/patient may be caught "off-guard" by the activation of the source of negative pressure 14.

This drawback of unpleasant noise due to automated pressure regulation may be more profound in dual lumen solutions as there are longer periods of time between activations of the source of negative pressure.

To this end, it is herein proposed to provide a "prepulse" to the user/patient, prior to activation of the source of negative pressure 14, in order to reduce the startle response exhibited by the user/patient due to the activation of the source of negative pressure 14. The startle response may also be referred to as acoustic startle reflex (ASR) herein.

This concept is generally referred to as prepulse inhibition (PPI) and may be understood as the ability of a low-intensity stimulus (i.e. the prepulse) to diminish the startle response elicited by a higher-intensity stimulus (i.e. the pulse). Here, the higher-intensity stimulus typically triggers the startle reflex of the subject. In general, the stimuli are acoustic, but tactile stimuli and light stimuli may also used. When the prepulse inhibition (PPI) is high, the corresponding one-time "startle response" is reduced. For the purpose of the present disclosure, the sound and/or vibrations emitted or generated by the source of negative pressure when activated or active is considered to form the "higher-intensity stimulus", i.e. the "pulse".

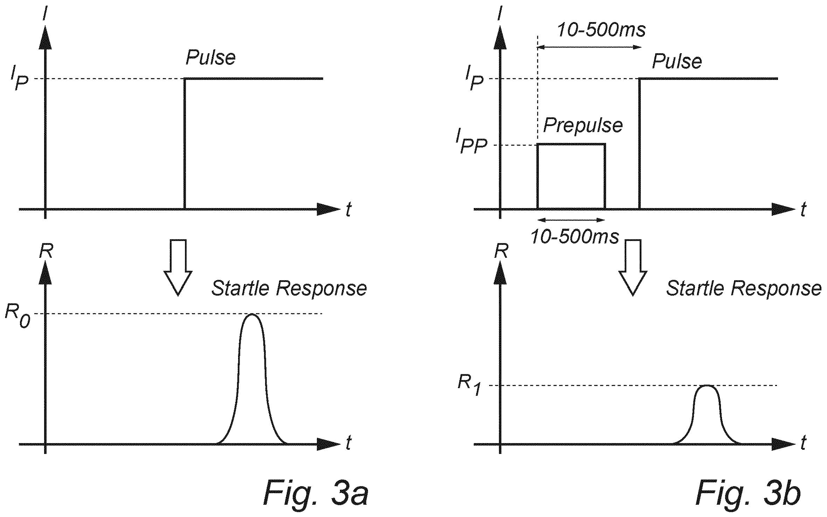

Figs. 3a-3b schematically illustrate the effects of prepulse inhibition on a subject's startle response, where Fig. 3a shows the startle response of a subject to a startle stimulus in the form of a pulse with intensity I_{P}, and Fig. 3b shows the startle response of a subject to the same startle stimulus but preceded by a prepulse of a lower intensity I_{PP}. As depicted in Figs. 3a and 3b, the startle response decreases (from R₀ to R₁) by introducing a prepulse. The pulse intensity *(I)* may for example be expressed in dB.

The reduction of the amplitude of startle response *(R)* reflects the ability of the nervous system to temporarily adapt to a stronger sensory stimulus when a preceding weaker signal is given to warn the subject or organism. Although the extent of the adaptation (i.e. startle response reduction) affects numerous systems of the subject, the most comfortable to measure are the muscular reactions.

Thus, in order to measure the prepulse inhibition one can measure the amplitude of the startle response for a set of subjects exposed to the pulse alone, and the amplitude of the startle response for another set of subjects exposed to the prepulse-plus-pulse. The percentage of the reduction in the amplitude of the startle response represents the prepulse inhibition. Stated differently, one can calculate the percentage of the magnitude of the PPI in accordance with the following formula: % prepulse inhibition = (100 - (100 × (startle amplitude on prepulse followed by pulse trial)/(startle amplitude on pulse trial alone))), i.e. % prepulse inhibition = (100 - (100 × R₁/R₀)).

The startle response *(R)* may as mentioned be measured by measuring muscular reactions, and the most common muscles used for measurements of the amplitude of startle response in humans, due to homogeneous and consistent electromyographic responses, are the facial, orbicularis oculi, and mentalis. Accordingly, the startle response may be measured by e.g. measuring the movements of oculomotor muscles ("eye-blink reflex" or "eye-blink response" assessed using electromyographic recording of orbicularis oculi muscle and by oculography).

Accordingly, the control circuitry 11 is further configured to generate a signal so to cause the apparatus 10 or an external handheld device 50 to emit a signal comprising an audible sound and/or a tactile signal to a user of the apparatus 10 during a time period prior to activation of the source of negative pressure 14. In the context of the present disclosure, the phrase "cause an apparatus to emit a sound" should be interpreted broadly, and encompasses embodiments where any suitable component or part comprised by the apparatus is the source of the emitted sound, as readily understood by the person skilled in the art.

In more detail, the control circuitry 11 may be configured to, in response to obtaining an indication of an activation of the source of negative pressure 14 to be performed, generate the signal so to cause the apparatus 10 or the external handheld device 50 to emit the signal to the user of the apparatus 10 during the time period, and activate the source of negative pressure 14 after expiry of the time period.

Thus, in some embodiments, the emitted signal is a first signal that defines a prepulse for the user of the apparatus 10 of a prepulse inhibition scheme, and the activation of the source of negative pressure 14 causes the apparatus 10 to emit a second signal comprising another audible sound and/or another tactile signal defining a pulse of the prepulse inhibition scheme. The term "prepulse inhibition scheme" may also be referred to as a "prepulse inhibiton protocol" and is mainly to be construed as a term for improving the readability of the present disclosure, and to elucidate that the control circuitry 11 of the apparatus 11 is configured to generate signals to enable prepulse inhibition for a user of the apparatus 10 in view of the noise and/or vibrations generated by the source of negative pressure 14.

The external handheld device 50 may for example be a mobile phone (e.g. a smartphone) operatively connected to the apparatus 10. Preferably, the external handheld device 50 is a personal device of the user of the wound treatment system 1, 1' that is generally worn by the user. The term "operatively connected" is in the context of the present disclosure to be understood as that the external handheld device 50 is configured to transmit and receive wireless signals 51 to and from the apparatus 10. Thus, in some embodiments, the apparatus 10 comprises suitable components (e.g. transceivers, antennas, filters, power amplifiers, etc.) and communication protocols in order to establish a connection with the external handheld device and to transmit and receive wireless signals 51 to and from the external handheld device.

Accordingly, in some embodiments, the apparatus 10 comprises a communication interface (not shown) connected to the control circuitry 11. The communication interface may comprise one or more antennas and one or more transceivers for transmitting and receiving wireless signals to and from the external handheld device 50. In some embodiments the communication interface comprises a Near Field Communication (NFC) interface. For example, the communication interface may include a Bluetooth^{®} radio (e.g. Bluetooth^{®} Low energy, BLE) or ZigBee^{®} radio, or any other suitable wireless technology for communicating 51 with the external handheld device 50.

In some embodiments, the time period starts 10 - 500 ms (milliseconds) prior to the activation of the source of negative pressure 14. In other words, the audible sound and/or the tactile signal is emitted during a time period that starts 10 - 500 ms prior to the activation of the source of negative pressure 14. Thus, the time period may start 20 ms, 40 ms, 60 ms, 80 ms, 100 ms, 120 ms, 180 ms, 240 ms, or 300 ms prior to the activation of the source of negative pressure. In some embodiments the source of negative pressure is activated immediately upon expiry of the time period. However, in some embodiments, the source of negative pressure is activated 1-500 ms after the expiry of the time period.

Moreover, in some embodiments, the time period is 10 - 500 ms long. In other words, the prepulse duration is a value within the range 10-500 ms. The time period (i.e. prepulse duration) may for example be 50 ms, 80 ms, 120 ms, 160 ms, 180 ms, 220 ms, or 300 ms. As readily understood by the skilled person in the art, the length of the time period, i.e. the duration of the prepulse, depends on when the time period starts in reference to the activation of the source of negative pressure 14. For example, if the time period starts 100 ms prior to the activation of the source of negative pressure 14, the maximum length of the time period is 100 ms. Accordingly, in some embodiments, the length of the time period is in the range between 10 ms and the time offset between the start of the time period and the activation of the source of negative pressure, where the time offset is in the range of 10 to 500 ms. In some embodiments, the time period starts 100-200 ms prior to the activation of the source of negative pressure, and the time period is a value within the range of 60-100 ms.

In some embodiments, the emitted signal comprises the audible sound, and the audible sound has a magnitude that is lower than a magnitude of an audible sound made by the source of negative pressure 14 while active. In other words, the prepulse comprises an audible sound that is lower in magnitude as compared to the sound made by the source of negative pressure 14 while active. In some embodiments, the magnitude of the prepulse is in the range of 20-50% of the magnitude of the audible sound made by the source of negative pressure 14 while active.

In some embodiments, the emitted signal (i.e. the prepulse) comprises an audible sound having a magnitude in the range of 50 - 90 dB. Furthermore, the audible sound (of the prepulse) may have a frequency in the range of 20 Hz - 20 000 Hz. In some embodiments, the prepulse comprises an audible sound having a magnitude that is at least 1 dB above an ambient background noise level. In some embodiments, the prepulse comprises an audible sound having a magnitude that is at least 1 dB higher than an ambient background noise level while being lower than a magnitude of the audible sound made by the source of negative pressure 14 while active.

The magnitude of the audible sound made by the source of negative pressure 14 while active may for example be determined by measuring the sound level in a test environment using one or more suitable sound transducers (e.g. microphones). The ambient background noise level may for example be predefined to a value in the range of 45-80 dB, such as e.g. 50 dB or 60 dB. However, in some embodiments, the apparatus 10 may further comprise a sound transducer (e.g. a microphone) connected to the control circuitry 11, where the sound transducer is configured to measure the ambient background noise level. Accordingly, the background noise level may be dynamically determined by the control circuitry 11 and the magnitude of the prepulse (in the form of an audible sound) may be dynamically set depending on the measured ambient background noise level. Thereby an improved prepulse inhibition may be achieved.

In some embodiments, the emitted signal comprises the audible sound, and the audible sound has an increasing sound magnitude profile during the time period. In other words, the audible sound may go from X dB at an initial portion of the time period to Y dB at an end portion of the time period, where Y > X. In some embodiments, the characteristics of the audible sound of the prepulse may be the same as the characteristics of the sound made by the made by the source of negative pressure 14 while active. The term "characteristics of a sound" may in the present context be understood as the frequency contents of a sound signal and their corresponding magnitudes, and their temporal context (i.e. the temporal order of sounds).

Further, in some embodiments, the emitted signal comprises the audible sound. The apparatus 10 may accordingly further comprise one or more loudspeakers 17 configured to output sound to the user of the apparatus. Moreover, the control circuitry 11 may be configured to control operation of the one or more loudspeakers 17, and the control circuitry may be further configured to control the one or more loudspeakers 17 so to emit the audible sound to the user of the apparatus 10 during the time period prior to activating of the source of negative pressure 14.

Still further, in some embodiments, the emitted signal comprises the tactile signal. The apparatus 10 may accordingly further comprise a vibrating device 18 configured to cause a vibration of the apparatus 10 upon activation of the vibrating device 18. Moreover, the control circuitry 11 may be configured to control operation of the vibrating device 18, and the control circuitry may be further configured to activate the vibrating device 18 so to emit the tactile signal to the user of the apparatus 10 during the time period prior to the activation of the source of negative pressure 14. The vibrating device 18 may comprise a tactile transducer, a motor, or a piezoelectric transducer.

Further, in reference to Fig. 2 and the dual lumen wound treatment system 1', in some embodiments, the apparatus 10 comprises an electronically controllable valve 40 configured to release negative pressure from the wound dressing 20 via an air lumen 41. Accordingly, in some embodiments the control circuitry 11 is configured to control an operation of the electronically controllable valve 40. The control circuitry 11 may be further configured to generate a signal so to cause the apparatus 10 or an external handheld device 50 to emit the signal comprising an audible sound and/or a tactile signal to the user of the apparatus 10 during a second time period prior to activation of the electronically controllable valve 40. In other words, the same principles of emitting a prepulse prior to the activation of the source of negative pressure 14 may be applied to the "flushing" process (i.e. the opening of the valve 40). Thereby any startle or discomfort to the user that is caused by the sound made by the valve 40 when releasing negative pressure may be reduced.

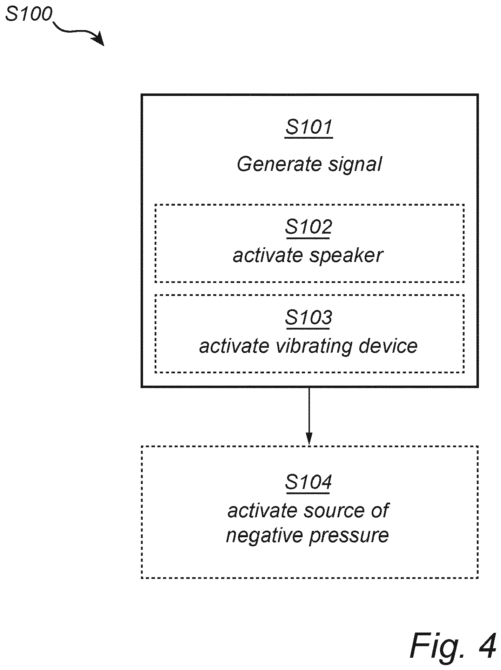

Fig. 4 is a schematic flowchart representation of a method S100 for controlling an apparatus comprising a source of negative pressure configured to provide negative pressure via a fluid flow path from an inlet of the source of negative pressure to a wound dressing. In other words, Fig. 4 is a schematic flowchart representation of a method S100 for controlling an apparatus of a wound treatment system 1, 1' according to any one of embodiments disclosed herein. The method S100 comprises generating S101 a signal so to cause the apparatus or an external handheld device to emit a signal comprising an audible sound and/or a tactile signal to a user of the apparatus during a time period prior to activation of the source of negative pressure.

In some embodiments, the method S100 comprises, in response to obtaining an indication of an activation of the source of negative pressure to be performed, generating S101 the signal so to cause the apparatus or the external handheld device to emit the signal to the user of the apparatus during the time period, and activating S104 the source of negative pressure after expiry of the time period.

In some embodiments, the emitted signal comprises the audible sound and the apparatus further comprises one or more loudspeakers configured to output sound to the user of the apparatus. The method S100 may accordingly further comprise causing S102 the one or more loudspeakers to emit the audible sound to the user of the apparatus during the time period prior to the activation S104 of the source of negative pressure.

In some embodiments, the emitted signal comprises the tactile signal and the apparatus further comprises a vibrating device configured to cause a vibration of the apparatus upon activation of the vibrating device. The method S100 may accordingly further comprise activating S103 the vibrating device so to emit the tactile signal to the user of the apparatus during the time period prior to the activation S104 of the source of negative pressure.

Executable instructions for performing these functions are, optionally, included in a non-transitory computer-readable storage medium or other computer program product configured for execution by one or more processors of an apparatus for providing reduced pressure to a wound dressing.

The herein disclosed technology has been presented above with reference to specific embodiments. However, other embodiments than the above described are possible and within the scope of the claims.

Different method steps than those described above, performing the method by hardware or software, may be provided within the scope of the claims. Thus, according to some embodiments embodiment, there is provided a non-transitory computer-readable storage medium storing one or more programs configured to be executed by one or more processors of an apparatus of a reduced-pressure wound treatment system, the one or more programs comprising instructions for performing the method according to any one of the above-discussed embodiments.

Generally speaking, a computer-accessible medium may include any tangible or non-transitory storage media or memory media such as electronic, magnetic, or optical media-e.g., disk or CD/DVD-ROM coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 11 (associated with the apparatus 10) may be or include any number of hardware components for conducting data or signal processing or for executing computer code stored in memory. The device 10 may accordingly have an associated memory, and the memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to some embodiments, any distributed or local memory device may be utilized with the systems and methods of this description. According to some embodiments embodiment the memory is communicably connected to the processor 11 (e.g., via a circuit or any other wired, wireless, or network connection) and includes computer code for executing one or more processes described herein.

It should be noted that any reference signs do not limit the scope of the claims, that some embodiments may be at least in part implemented by means of both hardware and software, and that several "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the appended claims. Likewise, software implementations could be accomplished with standard programming techniques with rule-based logic and other logic to accomplish the generating steps, activating steps, operating steps, causing steps, steps. The above mentioned and described embodiments are only given as examples and should not be limiting to the appended claims. Other solutions, uses, objectives, and functions within the scope of the below described patent claims should be apparent for the person skilled in the art.

## Claims

1. An apparatus (10) for providing reduced pressure to a tissue site, the apparatus (10) comprising:
a source of negative pressure (14) configured to provide negative pressure via a fluid flow path from an inlet of the source of negative pressure to a wound dressing (20);
control circuitry (11) configured to control operation of the source of negative pressure (14), the control circuitry (11) being further configured to:
generate a signal so to cause the apparatus (10) or an external handheld device (50) to emit a signal comprising an audible sound and/or a tactile signal to a user of the apparatus during a time period prior to activation of the source of negative pressure.

2. The apparatus (10) according to claim 1, wherein the emitted signal is a first signal that defines a prepulse for the user of the apparatus (10) of a prepulse inhibition scheme, and the activation of the source of negative pressure (14) causes the apparatus (10) to emit a second signal comprising another audible sound and/or another tactile signal defining a pulse of the prepulse inhibition scheme.

3. The apparatus (10) according to claim 1 or 2, wherein the time period starts 10 ms - 500 ms prior to the activation of the source of negative pressure (14).

4. The apparatus (10) according to any one of claims 1-3, wherein the control circuitry (11) is configured to:
in response to obtaining an indication of an activation of the source of negative pressure (14) to be performed:
generate the signal so to cause the apparatus (10) or the external handheld device (50) to emit the signal to the user of the apparatus during the time period;
activate the source of negative pressure (14) after expiry of the time period.

5. The apparatus (10) according to any one of claims 1-4, wherein the time period is 10-500 ms long.

6. The apparatus (10) according to any one of claims 1-5, wherein the emitted signal comprises the audible sound, and wherein the audible sound has a magnitude that is lower than a magnitude of an audible sound made by the source of negative pressure while active.

7. The apparatus (10) according to any one of claims 1-6, wherein the emitted signal comprises the audible sound, and wherein the audible sound has a magnitude in the range of 50-90 dB.

8. The apparatus (10) according to any one of claims 1-7, wherein the emitted signal comprises the audible sound, and wherein the audible sound has an increasing sound magnitude profile during the time period.

9. The apparatus (10) according to any one of claims 1-8, wherein the emitted signal comprises the audible sound, the apparatus (10) further comprising:
one or more loudspeakers (17) configured to output sound to the user of the apparatus (10);
wherein the control circuitry (11) is configured to control operation of the one or more loudspeakers (17), the control circuitry (11) being further configured to:
control the one or more loudspeakers (17) so to emit the audible sound to the user of the apparatus during the time period prior to activating of the source of negative pressure (14).

10. The apparatus (10) according to any one of claims 1-9, wherein the emitted signal comprises the tactile signal, the apparatus further comprising:
a vibrating device (18) configured to cause a vibration of the apparatus (10) upon activation of the vibrating device;
wherein the control circuitry (11) is configured to control operation of the vibrating device (18), the control circuitry (11) being further configured to:
activate the vibrating device (18) so to emit the tactile signal to the user of the apparatus during the time period prior to the activation of the source of negative pressure (14).

11. A method (S100) for controlling an apparatus (10) comprising a source of negative pressure (14) configured to provide negative pressure via a fluid flow path from an inlet of the source of negative pressure to a wound dressing (20), the method (S100) comprising:
generating (S101) a signal so to cause the apparatus or an external handheld device to emit a signal comprising an audible sound and/or a tactile signal to a user of the apparatus during a time period prior to activation of the source of negative pressure.

12. The method (S100) according to claim 11, further comprising:
in response to obtaining an indication of an activation of the source of negative pressure to be performed:
generating (S100) the signal so to cause the apparatus or the external handheld device to emit the signal to the user of the apparatus during the time period;
activating (S104) the source of negative pressure after expiry of the time period.

13. The method (S100) according to claim 11 or 12, wherein the emitted signal comprises the audible sound and wherein the apparatus further comprises one or more loudspeakers (17) configured to output sound to the user of the apparatus, the method (S100) further comprising:
causing (S102) the one or more loudspeakers to emit the audible sound to the user of the apparatus during the time period prior to the activation of the source of negative pressure.

14. A computer program product comprising instructions which, when the program is executed by a computing device of an apparatus for providing reduced pressure to a wound dressing, causes the apparatus to carry out the method (S100) according to any one of claims 11-13.

15. A computer-readable storage medium comprising instructions which, when executed by a computing device of an apparatus for providing reduced pressure to a wound dressing, causes the apparatus to carry out the method (S100) according to any one of claims 11-13.
